# EUROPEAN PATENT APPLICATION

(11) **EP 0 999 430 A1**
(43) Date of publication of application: **10.05.2000**
(21) Application number: 99420209.1
(22) Date of filing: 18.10.1999
(51) Int. Cl.: G01B 11/24, G06T 7/60, A61B 5/107

(54) **Three-dimensional contour measuring apparatus**

(30) Priority: 06.11.1998 FR 9814178
(71) Applicant: EASTMAN KODAK COMPANY, Rochester, New York 14650-2201 (US)
(72) Inventor: Philiponet, Etienne Pierre, 71102 Chalon Sur Saone Cedex (FR); Roger, Julien Serge Edmond, 61150 Montgaroult (FR)
(74) Representative: Buff, Michel

(57) **Abstract**

A process for recording data relating to an object in three dimensions to obtain a representation of said object, comprising the following steps:
a) illuminating, using a light beam in one main direction through a grid, at least one part of the object for obtaining a grid projection on said object;
b) recording at least one image of the part of the illuminated object by taking a shot using a recording device whose optical axis is arranged to form an angle less than or equal to 90° with the main direction of the light beam;
c) extracting the contours of the grid projection on the part of the object by recording curves representing the passages between the differences of luminance on the recorded image, the differences of luminance corresponding to the grid projection;
d) constructing, according to the grid used, the surface of the object from the curves obtained in c);
e) applying a texture corresponding to the texture of the object to the surface of the object.

## Description

The present invention relates to a process and system for retrieving data relating to an object in three dimensions to obtain a representation of the object so that, for example, it may be viewed from any angle, and in particular to obtain a representation of a face that can be distorted.

To obtain a representation of an object in three dimensions, a certain amount of data corresponding to the object has to be acquired first, and then the object in three dimensions has to be reconstructed from the data recovered.

U.S. Patent No. 4,825,263 describes a process for optically determining modifications in three dimensions of a face. The process includes illuminating the face through a squared structure according to two directions to obtain a squared representation of the face. The illuminated face is then viewed by means of a video camera to produce a video signal. The video signal is then converted into digital form and recorded. The squared representation of the face is then defined from the recorded video signal. Then the position of the coordinates of the intersection points of the squared representation are determined by optical correlation. An imagette representing two crossing lines is applied all over the image to determine the position of the intersections. Then the curvature of the intersection points is determined. The surface of the face can then be reconstructed in three dimensions from the coordinates and the curvature of the intersection points. Such a technique takes a relatively long time. Also, the imagette represents an intersection formed by two perpendicular straight lines. Whereas, the intersections sought are formed by curves somewhat distorted after the projection of the grid onto a face. Thus, they are not easy to mark. Such a process is not very robust.

An object of the invention is to provide for a process and system for retrieving data relating to an object in three dimensions to obtain a representation of the object.

It is also an object of the invention to obtain a representation of an object so that it can be viewed from any angle and be distorted.

The present invention relates to a process for retrieving data relating to an object in three dimensions to obtain a representation of the object. The process comprises the following steps:
a) illuminating, using a light beam in one main direction through a grid, at least one part of the object for obtaining a projection of the grid on the object;
b) recording at least one image of the part of the illuminated object by taking a shot using a recording device whose optical axis is arranged to form an angle less than or equal to 90° with the main direction of the light beam;
c) extracting the contours of the grid projection on the part of the object by recording curves representing the passages between the differences of luminance on the recorded image, the differences of luminance corresponding to the grid projection;
d) constructing, according to the grid used, the surface of the object from the curves obtained in c); and
e) applying a texture corresponding to the texture of the object to the surface of the object.

In accordance with another aspect of the present invention there is provided a system for retrieving data relating to an object in three dimensions for obtaining a representation of the object, the system comprising:
a light source which emits a light beam in one main direction;
a grid arranged between the light source and the object;
at least one recording device arranged so that its optical axis forms an angle less than or equal to 90° with the main direction of the light beam; and
a central processor that comprises means for extracting contours of the grid projection on a part of the object for obtaining curves and means for constructing a surface from the curves.

Other characteristics will appear on reading the following description, with reference to the drawings wherein:
Fig. 1 represents a diagram of a first embodiment of the device used to apply the invention process;
Fig. 2 represents the image obtained after the projection of the grid onto the object;
Fig. 3 represents an image having points corresponding to the contours of the object; and
Fig. 4 represents the model of the predefined object.

The invention process and system that will be described below is provided to retrieve data relating to an object in three dimensions to obtain a representation of the object, which can, for example, be viewed on a computer screen from any angle. The process and system enable the data to be retrieved so that, on the one hand, the surface of the object is recovered, and on the other hand, its texture can also be recovered. Using this recorded data, a user can, for example, distort one or more parts of the external surface of the object.

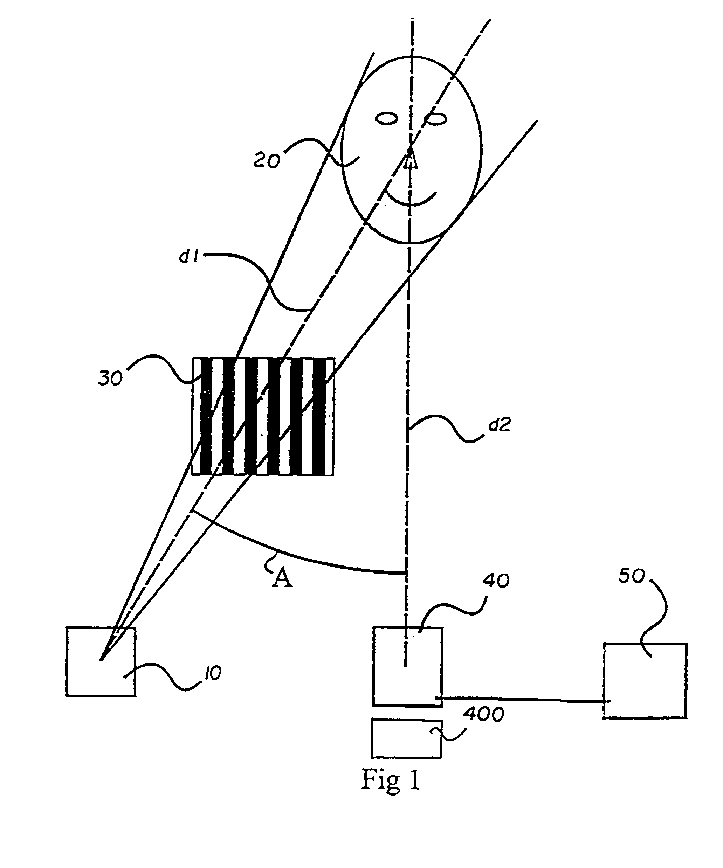

The device using the invention process can be seen by reference to Fig. 1. The device comprises a light source 10 provided to illuminate at least a part of an object 20. The light source 10 emits a light beam in one main direction dl. A structure 30 (or grid 30), is arranged between the light source 10 and the object 20. The position of the object 20 in relation to the structure or grid 30 and the light source depends, as will be seen below, on the size of the object 20 for which data is to be recovered. The structure 30 is, for example, an element with parallel bars. The use of a herringbone structure, a structure with concentric circles or any other motif can also be considered. The grid 30 is selected according to the precision of the data required to be recovered on the object 20. The more precise the representation of the object 20 is required, the smaller the pitch between two consecutive motifs of the grid 30. In preference a grid is used with parallel and vertical bars, that has dark zones and light zones of practically identical width. A recording device 40 is arranged for recording an image of the object 20 that is illuminated by the grid 30. The recording device is, for instance, a digital camera. Clearly a conventional camera can be used but a digital image is used for later processing of the image. In this case, for instance, a scanner can be used. The recording device 40 is arranged so that the direction of its optical axis d2 forms an angle A less than or equal to 90° with the main direction dl of the light beam. An angle of 60°, for example, can be selected. A central processor 50 is provided to process the data recorded by the recording device. The central processor 50 comprises means for extracting the contours of the grid projection on the part of the object for obtaining curves, and means for constructing a surface from the curves.

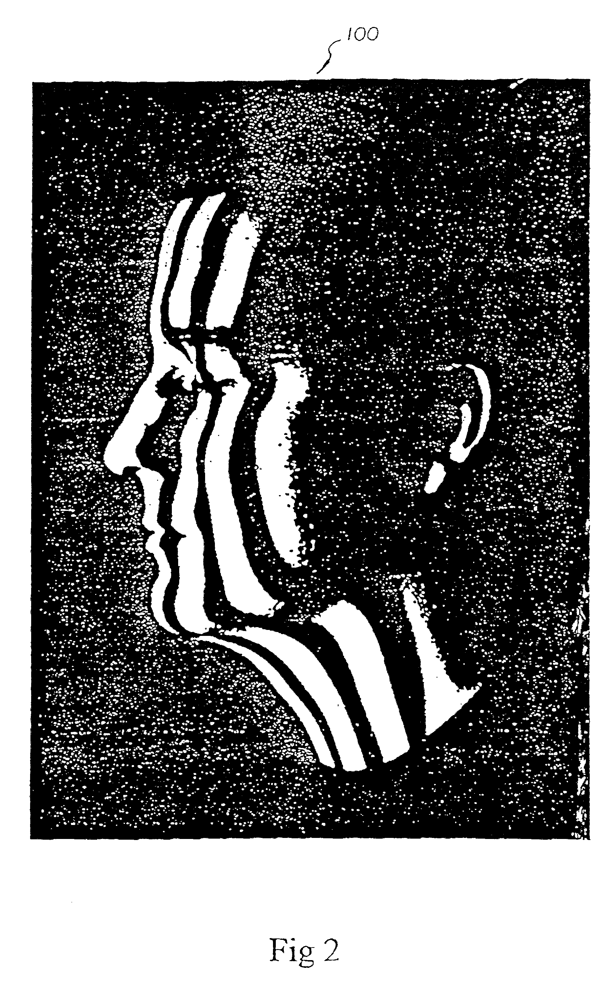

The invention process is provided, preferably, to be applied to an object 20 in the form of a head of an individual, and in particular to retrieve data relating to a face of the individual. In the following description, the head is considered to have a standard size. In order to record the data relating to the face, the object 20 in the form of a face is first illuminated using the light source 10, through the grid 30, for obtaining a projection of the grid on the face. In preference, the light source 10 is arranged opposite the face 20 in order to illuminate the whole face. As the recording device 40 is arranged with an angle A equal to about 60°, an image is recorded of a defined part of the head corresponding, in this embodiment, to about half the face, that is data between the nose and an ear. The recorded image 100 represented in Fig. 2 comprises bands of different luminance that correspond with the grid 30 projection. A file containing this digital image is then transmitted to the central processor 50 by means of which the data will be recovered.

To extract the contours of the grid projection on the face, a digital filter is used, for example, a gradient filter F of the type:

| | | | | |
|---|---|---|---|---|
| +a | -b | 0 | +b | -a |

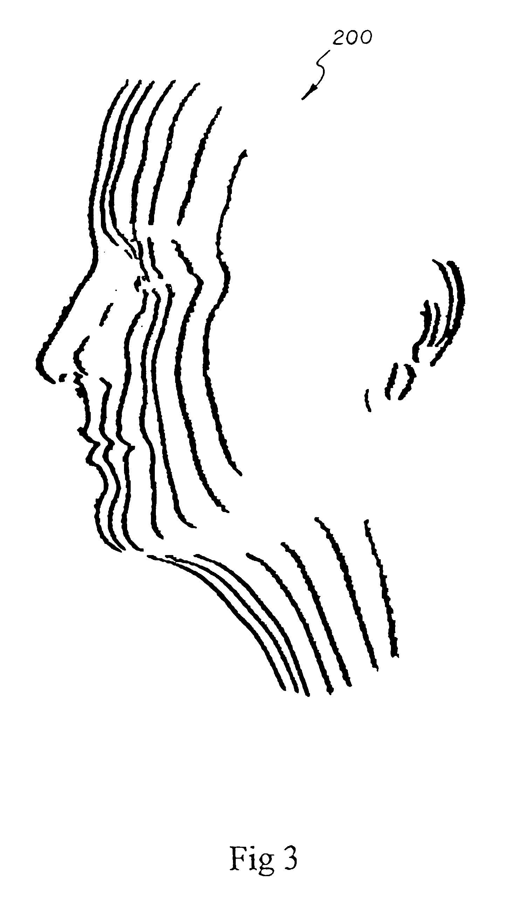

The filter enables the passage from the dark zones to the light zones and from the light zones to the dark zones to be determined. This filter F is applied to the whole image wherein each pixel has a gray level value, to obtain a new value for each pixel. A threshold is set above which the pixel is then considered to correspond to a passage between two zones of different brightness. For color images, a calculation well known to the skilled person in the art is performed to obtain a black and white image. Then one or more other filters are applied to eliminate noise and smooth the curves. Clearly the curves are recovered which correspond to the passages between the two zones of different brightness that are approximately in the direction of the grid. In this way an image 200 is obtained, represented in Fig. 3, which contains points that correspond to the contours of the grid projection on the defined part of the face, which has been recorded. The points form a certain number of mathematical curves that correspond to the grid projection on the face. When one or more curves are interrupted in certain zones of the face, for example, in the neck as can be seen in Fig. 3, this interruption is completed by linking the elements of the same curve using cubic smoothing.

The curves obtained are provided to construct the surface of the face taking into account the grid used. The step of reconstructing the surface of the face can be achieved using several methods. In the methods that will be described below, as the object 20 is a face, it will be assumed that it has an axis of symmetry that runs through the nose. Based on the recovered curves corresponding to the part of the face previously defined, that is half the face, the curves corresponding to the other half of the face are recovered by symmetry.

According to a first method, the surface of the face is reconstructed from the curves constituted by a set of pixels that have been recovered. These curves are then transformed into mathematical curves called "NURBS" (Non-Uniform Rational B-Splines) that comprise a certain number of control points which can be acted on to modify the curve. Preferably, these curves are processed before constructing a surface, this processing, including modifying the number of control points, their position and distribution. Then a NURBS surface of the face is constructed.

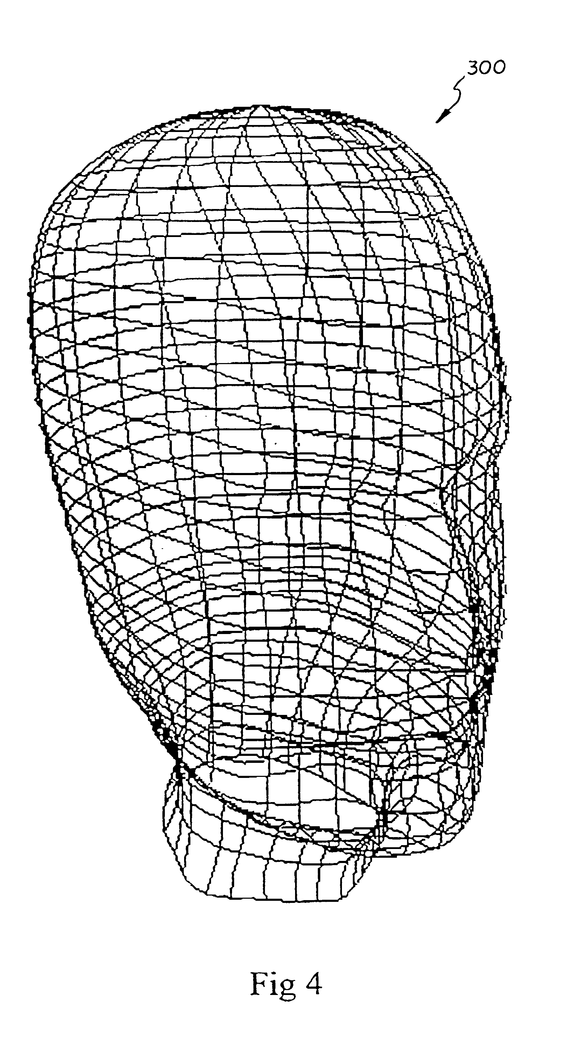

According to a second method, a standard model of the surface of the face is used. This standard model is constituted by a NURBS surface obtained by a program for modeling in three dimensions known to the skilled person in the art. The standard model contains NURBS curves whose direction corresponds approximately with the direction of the grid projection on the object. In the embodiment used, a standard head model 300 is defined as represented in Fig. 4. The size of the standard model corresponds to a standard head size. The number N of NURBS curves whose direction corresponds approximately with the grid projection between the nose and an ear, should equal the number of curves recovered after projection of the grid on the defined part of the face of a standard head. Clearly when the retrieval of data relating to a much smaller head is required, the head is positioned, when shooting, so that the grid projection on the part of the face is such that N curves are recovered after projection of the grid on the defined part of the face, that is between the nose and an ear. The NURBS curves of the model are drawn towards the curves of the image of the defined part of the face that have been recovered, by acting on the control points of the NURBS curves. The first curve recovered from the face is considered to pass through the tip of the nose. To start with the model curve that passes through the tip of the nose is made to correspond with the curve from the face. Thus, the position of the control points of this curve are modified to make it correspond to the face curve. Then in the same way each curve contiguous with the model curve previously processed, is processed. For each curve, several series of adjustments may be necessary. The starting model of the face surface is thus distorted to obtain a representation of the face surface.

According to a third method, NURBS curves that generate a surface of a part of a head, usually the rear, are used, and the recovered curves are added having been transformed into NURBS that will generate the surface of the other part of the head, that is the face. Then a NURBS surface of the head can be created.

It is clear that in the methods described above, a polygonal surface can be created from the recovered curves rather than creating a NURBS surface. A polygonal model can also be calculated from the NURBS surface obtained.

Several shots through the grids can be taken to recover data corresponding to unconnected parts of the face. The various shots are taken successively, and for each shot, the whole invention process is applied. Thus the whole surface of the face can be reconstructed. However, if the object is symmetrical, as for instance in the case of a face, a single shot of one side of the face can be taken. The other side is constructed by extrapolation.

A representation in three dimensions is then obtained but it does not include the texture. It is then just a matter, for example, of applying a standard texture of the object to the representation. Parts of the object, for instance in the case of a head, the hair, can be retrieved using a form and a standard texture.

According to one embodiment of the invention, the process further comprises a second shot of the object using a second recording device 400 (Fig. 1), without this time projecting the grid. Preferably, this shot is taken to record the whole face of the object. This shot then allows the object's texture to be recovered. This texture is then applied to the representation. This is achieved by again using the particular marked point of the object, for example, the nose in the case of the face, and the texture is positioned on the representation of the face. The representation obtained has high definition.

According to another embodiment, the object is lit with radiation having a wavelength in a first spectrum, for example, visible light radiation. A grid can also be projected with radiation having a wavelength in a second wavelength spectrum different from the first spectrum, for example, in the infrared. In this embodiment, the recording device 40 provided to record the grid projection on the object includes a sensor sensitive solely to the radiation of wavelength belonging to the second spectrum. The second recording device 400 provided to record the texture of the object includes a sensor sensitive solely to the radiation of wavelength belonging to the first spectrum. Two simultaneous shots are taken, one allowing the recording of a part of the object with the grid projection, and the other allowing the recording of the object's texture without the grid projection.

Clearly it is possible to record the texture and the contours of the grid projection on the object in a single recording at the same time, and to recover this data by using algorithms that allow for the object, grid and light source, at the same time thus enabling simultaneous recording.

The invention as described above enables a representation of an object in three dimensions to be obtained, which comprises on the one hand, the object's surface, and on the other hand, its texture.

Such a representation can be used for distortion purposes by, for example, an image distortion program. In the case of the face, it is possible, for example, to enlarge one part while keeping an exact surface of the whole face.

## Claims

1. A process for recording data relating to an object in three dimensions to obtain a representation of said object, comprising the following steps:
a) illuminating, using a light beam in one main direction through a grid, at least one part of the object for obtaining a grid projection on said object;
b) recording at least one image of the part of the illuminated object by taking a shot using a recording device whose optical axis is arranged to form an angle less than or equal to 90° with the main direction of the light beam;
c) extracting the contours of the grid projection on the part of the object by recording curves representing the passages between the differences of luminance on the recorded image, the differences of luminance corresponding to the grid projection;
d) constructing, according to the grid used, the surface of the object from the curves obtained in c);
e) applying a texture corresponding to the texture of the object to the surface of the object.

2. A process according to Claim 1 wherein step d) is obtained by drawing the curves, belonging to a standard model of the surface of the object whose direction corresponds approximately with the direction of the grid projection on the object, towards the curves obtained in c) to distort a part of the model.

3. A process according to Claim 1 wherein step d) is obtained by adding the curves obtained in c) to a standard model of the object comprising standard curves.

4. A process according to one of the previous claims wherein, if the part of the object has a symmetrical part in the object, the part of the curves obtained in c) is extrapolated to the symmetrical part.

5. A process according to Claim 1 wherein several unconnected parts of the object are illuminated successively according to step a), and steps b), c) and d) are applied for each part.

6. A process according to one of the previous claims wherein a second shot of the part of the object is taken, without projecting the grid, to record the object's texture.

7. A process according to Claim 6 wherein the texture applied in step e) is that recorded by the second shot.

8. A process according to one of the previous claims that further comprises the step consisting in recording a representation corresponding to the surface of the object to which a texture has been applied to obtain an image of the object.

9. A process according to Claim 8 that further comprises the step of distorting the image of the object.

10. A process according to any one of the previous claims wherein the object is a face.

11. A system for retrieving data relating to an object in three dimensions for obtaining a representation of said object (20) which comprises:
- a light source (10) emitting a light beam in one main direction.
- a grid (30) arranged between the light source and the object (20);
- at least one recording device (40) arranged so that its optical axis forms an angle less than or equal to 90° with the main direction of the light beam;
- a central processor (50) that comprises means for extracting the contours of the grid projection on a part of the object for obtaining curves and means for constructing a surface from the curves.

12. A system according to Claim 11 that further comprises a digital model of a representation in three dimensions of the object (20).

13. A system according to Claim 11 or 12 that further comprises a second recording device (400).

14. A system according to Claim 13 wherein the at least one recording device (40) comprises a sensor sensitive solely to wavelengths belonging to a first spectrum, and the second recording device is sensitive solely to wavelengths belonging to a second spectrum different from the first, and wherein the light source emits radiation of wavelength in the first spectrum, and the grid is projected on the object with a radiation of wavelength in the second spectrum.
